# EUROPÄISCHE PATENTSCHRIFT

(11) **EP 1 385 928 B1**
(45) Veröffentlichungstag und Bekanntmachung des Hinweises auf die Patenterteilung: **25.01.2006**
(21) Anmeldenummer: 02764066.3
(22) Anmeldetag: 22.04.2002
(51) Int. Cl.: C10M 159/16, C07C 37/84, C07C 213/10, C07C 215/50, C07C 39/06

(54) **VERFAHREN ZUR REINIGUNG VON LANKETTIGEN ALKYLPHENOLEN UND MANNICH-ADDUKTEN DAVON**
METHOD FOR PURIFYING LONG-CHAIN ALKYL PHENOLS AND THE MANNICH ADDUCTS THEREOF
PROCEDE DE PURIFICATION D'ALKYLPHENOLS A LONGUE CHAINE ET PRODUITS D'ADDITION DE MANNICH DESDITS ALKYLPHENOLS

(30) Priorität: 23.04.2001 DE 10119738
(43) Veröffentlichungstag der Anmeldung: 04.02.2004
(73) Patentinhaber: BASF Aktiengesellschaft, 67056 Ludwigshafen (DE)
(72) Erfinder: LANGE, Arno, 67098 Bad Dürkheim (DE); RATH, Hans, Peter, 67269 Grünstadt (DE); WALTER, Marc, 67227 Frankenthal (DE)
(74) Vertreter: Pohl, Michael Friedrich
(86) Internationale Anmeldenummer: PCT/EP2002/004415
(87) Internationale Veröffentlichungsnummer: WO 2002/086037

(56) Entgegenhaltungen:
- US-A- 3 368 972
- US-A- 3 904 595

## Beschreibung

Die vorliegende Erfindung betrifft ein Verfahren zur Reinigung von Polyisobutenylphenolen mit einem zahlenmittleren Molekulargewicht von 200 bis 4000 und Mannich-Addukten davon.

Aminoalkylierte Polyalkylenphenole, wie sie durch Mannich-Reaktion von Aminen und Aldehyden mit Polyalkylen-substituierten Phenolen zugänglich sind, spielen eine wichtige Rolle als Kraftstoffadditive zur Reinhaltung von Ventilen und Vergaser bzw. Einspritzsystemen von Ottomotoren (vgl. z. B. M. Rossenbeck in Katalysatoren, Tenside, Mineralöladditive, Hrsg. J. Falbe, U. Hasserodt, S. 223, G. Thieme Verlag, Stuttgart 1978).

Die rohen Umsetzungsprodukte der Mannich-Reaktion zeigen oft schwankende Reinigungswirkung und sind mit einer Reihe von Nachteilen behaftet. Aufgrund der komplexen Zusammensetzung der Gemische beobachtet man oft eine dunkle Färbung und einen intensiven Geruch, die die Kundenakzeptanz negativ beeinflussen. Vielfach ist auch die Viskosität bei niederen Temperaturen zu hoch, was zum sogenannten Ventilstecken führen kann. Darunter versteht man den vollständigen Kompressionsverlust auf einem oder mehreren Zylindern des Verbrennungsmotors, wenn die Federkräfte nicht mehr ausreichen, die Ventile ordnungsgemäß zu schließen.

Ursache für die unerwünschten Eigenschaften sind vielfach Nebenprodukte und niedermolekulare Anteile, die von der Herstellung des Alkylphenols herrühren. Die als Edukt eingesetzten Alkylphenole werden meist durch Alkylierung von Phenol mit Polyolefinen, wie Polyisobuten, hergestellt. Bei der Alkylierung mit Polyisobuten wird vielfach eine Erniedrigung des Molekulargewichts, d. h. eine teilweise Spaltung der Polyisobutenkette, beobachtet. Das abgespaltene Isobuten kann niedere Alkylphenole, wie tert-Butylphenol oder tert-Octylphenol, bilden.

Ebenso können sich bei der Mannich-Reaktion, insbesondere mit primären Aminen und Formaldehyd, störende Nebenprodukte bilden.

Die US 3,368,972 beschreibt eine Motoröladditivzusammensetzung, die Mannich-Addukte von Alkyl-substituierten hydroxy-aromatischen Verbindungen enthält. Als geeignete hydroxy-aromatische Verbindungen werden Polypropylphenol, Polybutylphenol und Polyamylphenol genannt. In Beispiel 1 dieses Dokuments wird das Reaktionsgemisch mit Dimethylamin und Formaldehyd zur Entfernung unumgesetzten Amins und Formaldehyds mit Toluol und Butylalkohol versetzt und dreimal mit heißem Wasser gewaschen.

Die US 3,904,595 beschreibt Dispergatoren für Motoröle, die Mannich-Addukte von Alkylphenolen und Schwefel enthalten. Beispiel 2 dieses Dokuments beschreibt die Herstellung eines Polybutylphenols aus Phenol und Polybuten in Gegenwart von BF₃-Phenolat als Katalysator. Zur Aufarbeitung wird das Reaktionsgemisch mit Methanol und Ammoniak versetzt und unter Erwärmen gerührt. Die resultierende organische Phase wird nochmals mit Methanol gewaschen.

Der vorliegenden Erfindung liegt die Aufgabe zugrunde, ein einfaches Verfahren zur Entfernung von Nebenprodukten und niedermolekularen Anteilen aus langkettigen Alkylphenolen und deren Mannich-Addukten bereitzustellen.

Erfindungsgemäß wird diese Aufgabe durch ein Verfahren zur Reinigung eines unter Polyisobutenylphenolen mit einem zahlenmittleren Molekulargewicht von 200 bis 4000 oder Mannich-Addukten davon ausgewählten Phenolderivats gelöst, bei dem man das Phenolderivat mit einem Extraktionsmittel einer Polarität E_{T}(30) von 57 bis 38 kcal/mol, vorzugsweise 56 bis 48 kcal/mol, innig in Kontakt bringt, eine Phenolderivat-haltige Phase und eine Extraktionsmittelphase sich voneinander trennen lässt und die Extraktionsmittelphase entfernt.

Dem erfindungsgemäßen Verfahren kann einerseits ein Polyisobutenylphenol unterworfen werden; das gereinigte Polyisobutenylphenol kann dann zu einem Mannich-Addukt umgesetzt werden, wie dies weiter unten beschrieben ist. Alternativ kann man ein rohes Polyisobutenylphenol zu einem Mannich-Addukt umsetzen und das Mannich-Addukt nach dem erfindungsgemäßen Verfahren reinigen.

Das Phenolderivat kann als solches, d. h. in Substanz, dem erfindungsgemäßen Verfahren unterworfen werden. In vielen Fällen ist es zur Verringerung der Viskosität von Vorteil, das Phenolderivat in einem unpolaren Lösungsmittel, d. h. einem solchen einer Polarität E_{T}(30) von 35 bis 30 kcal/mol, zu lösen und die Lösung mit dem Extraktionsmittel in Kontakt zu bringen. Das unpolare Lösungsmittel und die auf das Phenolderivat bezogene relative Menge des unpolaren Lösungsmittels werden so gewählt, dass bei der Temperatur, bei der das erfindungsgemäße Verfahren durchgeführt wird, die Lösung des Phenolderivats und das Extraktionsmittel nicht vollständig mischbar sind. Mit anderen Worten weisen sie eine Mischungslücke auf, so dass nach dem Inkontaktbringen eine spontane Entmischung in eine Phenolderivat-haltige polare Phase und eine Extraktionsmittelphase erfolgt. Geeignete unpolare Lösungsmittel sind aliphatische oder aromatische Kohlenwasserstoffe, wie n-Pentan, n-Hexan, n-Heptan, n-Octan sowie deren Isomere, Petrolether, Ligroin, Benzol, Toluole, Xylole oder kommerzielle Lösungsmittelgemische, wie Solvesso® oder Aromatics® 150, sowie Gemische davon.

Die Lösung weist im Allgemeinen einen Gehalt an Phenolderivat von wenigstens 5 Gew.-% auf, meist 20 bis 90 Gew.-%.

Da solche Lösungsmittel auch bei der Herstellung der Phenolderivate eingesetzt werden, kann auch das rohe Reaktionsgemisch direkt der Extraktion unterworfen werden.

Im erfindungsgemäßen Verfahren wird als Extraktionsmittel ein Lösungsmittel mit einer Polarität E_{T}(30) von 57 bis 38 kcal/mol, vorzugsweise 56 bis 48 kcal/mol, verwendet. Die Bestimmung der Lösungsmittelpolarität anhand der E_{T}(30)-Skala ist in Liebigs Ann. Chem. 1983, 721-743, beschrieben, worauf vollinhaltlich Bezug genommen wird. Es wurde gefunden, dass Lösungsmittel der angegebenen Polarität ein hohes Lösevermögen für die Nebenprodukte und niedermolekularen Anteile aufweisen, mit dem Phenolderivat aber nicht mischbar sind, so dass sie eine eigene Phase bilden, in der sich die Nebenprodukte und niedermolekularen Anteile anreichern und die leicht entfernt werden kann. Ist die Polarität des Extraktionsmittels dagegen niedriger als angegeben, so zeigt es keine zufriedenstellende Phasentrennung mit dem Phenolderivat. Ist die Polarität höher als angegeben, ist das Lösevermögen des Extraktionsmittels für die zu entfernenden Komponenten nicht ausreichend.

Je nach Art des verwendeten Extraktionsmittels und der Alkylkettenlänge des Alkylphenols oder seines Mannich-Addukts ist die Extraktionsmittelphase oder die Phenolderivat-haltige Phase die schwerere Phase. Die sich abtrennende Extraktionsmittelphase ist, im Allgemeinen an der intensiven dunklen Färbung erkennbar, die von den extrahierten farbigen Nebenprodukten herrührt.

Für das erfindungsgemäße Verfahren geeignete Extraktionsmittel sind vor allem den Substanzklassen der Alkohole, wie Methanol, Ethanol, n-Propanol oder Isopropanol, Nitrile, wie Acetonitril, Ketone, wie Aceton oder Butanon, oder Ester, wie Essigester, oder Butylacetat, Lactone, wie Butyrolacton, Amide, wie Formamid oder Dimethylformamid, zuzuordnen. Bevorzugte Extraktionsmittel sind Methanol, Ethanol, n-Propanol, Isopropanol, Aceton oder Butanon.

Ebenso sind Gemische der Lösungsmittel untereinander oder mit Wasser geeignet, z. B. in einer Menge von bis zu 20 Gew.-%. Ein geeignetes Extraktionsmittel ist z. B. Methanol mit 10 Gew.-% Wasser.

Im Allgemeinen verwendet man 1 bis 200 Gew.-Teile, vorzugsweise 20 bis 100 Gew.-Teile, Extraktionsmittel auf 100 Gew.-Teile Phenolderivat oder dessen Lösung in einem unpolaren Lösungsmittel.

Das Inkontaktbringen kann kontinuierlich oder diskontinuierlich durchgeführt werden. Mehrere diskontinuierliche Operationen können kaskadenartig hintereinander durchgeführt werden, wobei die von der Extraktionsmittelphase abgetrennte Phenolderivat-haltige Phase jeweils mit einer frischen Portion Extraktionsmittel in Kontakt gebracht wird. Zur diskontinuierlichen Durchführung bringt man unter mechanischer Bewegung, z. B. durch Rühren oder Schütteln, das Phenolderivat oder seine Lösung in einem unpolaren Lösungsmittel und das Extraktionsmittel in einem geeigneten Gefäß in Kontakt, lässt das Gemisch zur Phasentrennung ruhen und entfernt eine der Phasen, indem man zweckmäßigerweise die schwerere Phase am Boden des Gefäßes abzieht.

Zur kontinuierlichen Durchführung führt man das Extraktionsmittel vorzugsweise dem Phenolderivat in einer Extraktionszone entgegen. Hierfür benutzt man mit Vorteil vertikal angeordnete Trennsäulen, die zur Schaffung einer hohen Phasenaustauschfläche gewöhnlich mit Füllkörpern bzw. bewegten oder unbewegten Einbauten versehen sind. Die schwere Phase wird vorzugsweise im oberen Bereich der Trennsäule, die leichtere Phase im unteren Bereich der Trennsäule zugeführt und so einander entgegen geführt. Die leichte Phase setzt sich am Kopf der Trennsäule auf der schweren ab und kann durch ein Überlaufrohr entfernt werden. Die schwere Phase sammelt sich am Boden der Trennsäule und kann, vorzugsweise über ein Steigrohr, abgeführt werden. Dem Fachmann sind alternative Ausführungsformen einer kontinuierlichen Flüssig-Flüssig-Extraktion bekannt.

Während des Inkontaktbringens kann die Phenolderivat-haltige Phase zerteilt und die Extraktionsmittelphase unzerteilt oder umgekehrt vorliegen.

Der Erfolg des erfindungsgemäßen Reinigungsverfahrens kann insbesondere bei kontinuierlicher oder kaskadenartiger Durchführung überwacht werden, wenn man im verbrauchten Extraktionsmittel die Konzentration der extrahierten Bestandteile bestimmt. Dies kann durch Messen von Dichte oder Brechungsindex erfolgen. Ebenso können verfahren der IR- oder UV-VIS-Spektroskopie, gegebenenfalls in einer "on-line"-Methode, herangezogen werden. Man kann das Reinigungsverfahren z. B. abbrechen, wenn die Konzentration unter einen vorgegebenen Wert gesunken ist. Das verbrauchte Extraktionsmittel kann aufgearbeitet werden, z. B. durch Destillation oder Membranverfahren. Hierbei kann es in nicht umgesetzte Einsatzstoffe, wie Phenole, die in das Herstellungsverfahren zurückgeführt werden können, und Nebenprodukte, die verworfen werden, aufgetrennt werden. Durch das erfindungsgemäße Verfahren ist eine Abreicherung von Nebenprodukten und niedermolekularen Anteilen im Phenolderivat um einen Faktor von mehr als 10, insbesondere mehr als 20, auf einfache Weise möglich.

Das erfindungsgemäße Verfahren erfolgt im Allgemeinen bei einer Temperatur von 0 bis 120 °C, vorzugsweise 10 bis 60 °C, z. B. bei Raumtemperatur. Man arbeitet im Allgemeinen bei Normaldruck, wobei ein Arbeiten bei Überdruck ebenfalls möglich ist.

Polyisobutenylphenole, die nach dem erfindungsgemäßen Verfahren gereinigt werden können, werden im Allgemeinen durch Umsetzung eines Phenols mit einem im Wesentlichen einfach ungesättigten Olefin geeigneter Kettenlänge in Gegenwart eines Alkylierungskatalysators erhalten. Als Phenol sind (unsubstituiertes) Phenol selbst oder substituierte Phenole geeignet. Die substituierten Phenole weisen vorzugsweise neben der Hydroxylgruppe einen oder zwei weitere Substituenten auf, die sich vorzugsweise in ortho-Position zur Hydroxylgruppe befinden. Geeignete Substituenten sind z. B. C₁-C₄-Alkyl oder C₁-C₄-Alkoxy. Besonders bevorzugt sind Phenol, 2-Methylphenol und 2-Ethylphenol.

Erfindungsgemäß werden Polyisobutene, insbesondere sogenannte "hochreaktive Polyisobutene, die einen Anteil an Isobuten-Einheiten von mehr als 80 Mol-%, insbesondere mehr als 90 Mol-%, aufweisen eingesetzt. Die Polyisobutene weisen vorzugsweise ein zahlenmittleres Molekulargewicht im Bereich von 500 bis 1500 und eine Polydispersität zwischen 1,1 und 5,0, vorzugsweise weniger als 1,9, auf. Unter Polydispersität versteht man den Quotienten des gewichtsmittleren Molekulargewichts M_{w} durch das zahlenmittlere Molekulargewicht Mₙ.

Die Alkylierung erfolgt geeigneterweise bei einer Temperatur von -10 bis 80 °C, vorzugsweise 0 bis 40 °C. Geeignete Alkylierungskatalysatoren sind Protonensäuren, wie Schwefelsäure, Phosphorsäure und organische Sulfonsäuren, z. B. Trifluormethansulfonsäure; Lewissäuren, wie Aluminiumtrihalogenide, z. B. Aluminiumtrichlorid oder Aluminiumtribromid, Bortrihalogenide, z. B. Bortrifluorid und Bortrichlorid, Zinnhalogenide, z. B. Zinntetrachlorid, Titanhalogenide, z. B. Titantetrabromid und Titantetrachlorid, und Eisenhalogenide, z. B. Eisentrichlorid. Lewissäure-Alkylierungskatalysatoren können in Kombination mit Elektronendonoren, wie Alkoholen, insbesondere C₁-C₆-Alkanolen, Phenolen oder Ethern, verwendet werden. Besonders bevorzugt sind Bortrifluoridetherat und Bortrifluoridphenolat.

Geeignete Lösungsmittel für die Alkylierung sind Kohlenwasserstoffe, insbesondere aliphatische Kohlenwasserstoffe, wie Pentan, Hexan und Heptan, Petroleumbenzine mit Siedebereichen zwischen 35 und 100 °C, Dialkylether, z. B. Diethylether, und halogenierte Kohlenwasserstoffe, wie Dichlormethan oder Trichlormethan. Ebenso können Aromaten, wie Toluol, xylol, und deren Isomere eingesetzt werden.

Zur Herstellung der Mannich-Addukte wird das Alkylphenol mit einem Aldehyd und einem primären oder sekundären Amin umgesetzt. Geeignete Verfahren sind z. B. in der US-A 4,231,759;
US-A 4,117,011; US-A 5,634,951; US-A 5,725,612; GB 1,368,532 oder den älteren Anmeldungen DE 199 48 114.8 und DE 199 48 111.3 beschrieben.

Als Aldehyd wird vorzugsweise Formaldehyd umgesetzt. Geeignete Formaldehydquellen sind wässrige Formaldehydlösung und Formaldehydoligomere, wie Trioxan und Paraformaldehyd.

Geeignete primäre Amine sind beispielsweise Methylamin, Ethylamin, n-Propylamin, Isopropylamin, Dimethylaminopropylamin, Anilin und Benzylamin. Geeignete sekundäre Amine sind Dimethylamin, Diethylamin, Piperidin, Morpholin und Piperazin. Weiterhin geeignet sind Alkylendiamine, Dialkylentriamine, Trialkylentetramine und Polyalkylenpolyamine, wie Oligo- oder Polyalkylenimine, insbesondere Oligo- oder Polyethylenimine, vorzugsweise solche aus 2 bis 20 Ethylenimineinheiten. Außerdem eignen sich die Umsetzungsprodukte von Alkylenoxiden, insbesondere Ethylenoxid, mit primären Aminen.

Das bei der Mannich-Reaktion gebildete Reaktionswasser lässt sich durch Erhitzen in Gegenwart eines Schleppmittels entfernen. Geeignete Schleppmittel sind Benzol, Toluol, Xylol und Gemische davon.

Alkylphenol, Aldehyd und Amin werden vorzugsweise in einem molaren Verhältnis von 1 : 0,5 : 0,5 bis 1 : 4 : 4 eingesetzt.

Obgleich die unterschiedlichsten Polyisobutenylphenole und deren Mannich-Addukte nach dem erfindungsgemäßen Verfahren gereinigt werden können, ist es für 4-Polyisobutenylphenol und dessen Mannich-Addukte besonders geeignet.

Das nach dem erfindungsgemäßen Verfahren gereinigte Mannich-Addukt oder ein aus einem nach dem erfindungsgemäßen Verfahren gereinigten Phenolderivat hergestelltes Mannich-Addukt eignet sich als Tensid oder oberflächenaktive Substanz, z. B. als Detergenzadditiv in Kraft- und Schmierstoffzusammensetzungen, gegebenenfalls in Kombination mit weiteren üblichen Kraft- und Schmierstoffadditiven.

Als Beispiel für solche zusätzlichen Komponenten sind Polyalkenmono- oder Polyalkenpolyamine, Nitropolyisobutane, Hydroxynitropolyisobutane, Copolymere von C₂-C₄₀-Olefinen mit Maleinsäureanhydrid, deren Carboxylgruppe ganz oder teilweise neutralisiert sind, Sulfobernsteinsäurealkylester und Salze davon, Tridecanol-oder Isotridecanolbutoxylate, Isononylphenolbutoxylate, Adipate, Phthalate, Isophthalate, Terephthalate und Trimellitate des Isooctanols, Isononanols, Isodecanols und Isotridecanols, Polypropylenoxid und Polybutylenoxid sowie deren Derivate.

Die Erfindung wird durch die folgenden Beispiele näher veranschaulicht.

### Beispiel 1

Man erwärmte 1200 g 4-Polyisobutenylphenol (Mₙ Polyisobuten = 1000) in 500 ml Xylol mit 97 g Formaldehydlösung (37 Gew.-%) und 135 g Dimethylaminlösung (40 Gew.-%) 3 Stunden unter Rückfluss und Auskreisen von Wasser zum Sieden. Die abgekühlte Reaktionslösung wurde dann mit 500 ml Methanol verrührt. Nach der Phasentrennung wurde die Methanolphase abgetrennt und verworfen. Dieser Vorgang wurde zweimal wiederholt. Die Produktphase wurde schließlich am Rotationsverdampfer bei 140 °C und 10 mbar eingeengt. Man erhielt 1240 g 2-(Dimethylaminomethyl)-4-polyisobutenylphenol als helles Öl.

### Beispiel 2

Eine Mannichbase wurde wie in Beispiel 1 aus einem Polyisobutenylphenol (Mₙ Polyisobuten = 1000), Formaldehyd und Dimethylamin im molaren Verhältnis 1:1,1:1,1 hergestellt. Das Xylol wurde durch Abdampfen entfernt (ohne vorhergehende Wäsche mit Methanol). 100 g der Mannichbase wurden 30 Minuten mit 100 ml Methanol verrührt. Man trennte die Produktphase ab und entfernte Restmengen an Methanol am Rotationsverdampfer bei 100 °C und 20 mbar. Es wurden 94 g gereinigtes Produkt erhalten.

In weiteren Versuchen wurden anstelle des Methanols 100 ml der nachstehenden Lösungsmittel mit 100 g Mannichbase verwendet. Die Ausbeute an gereinigtem Produkt ist in Klammern angegeben.

Lösungsmittel: Ethanol (91 g), Aceton (89 g), Ethylacetat (90 g), Dimethylformamid (95 g).

### Beispiel 3

Ein 4-Polyisobutenylphenol wurde aus 225 g Phenol (gelöst in 130 ml Toluol), 14 g BF₃-Phenolat sowie 1200 g Polyisobuten (Mₙ = 1000; gelöst in 700 ml Hexan) hergestellt. Nach der Reaktion wurde das Rohgemisch mit 500 ml Methanol extrahiert und die Methanolphase wurde abgetrennt. Dieser Vorgang wurde dreimal wiederholt. Die Lösung des Polyisobutenylphenols wurde am Rotationsverdampfer bei 140 °C und 10 mbar eingeengt. Man erhielt 1250 g 4-Polyisobutenylphenol als helles Öl. Die Methanolphasen wurden an einer 100 cm langen Füllkörperkolonne mit Edelstahl-Maschendrahtwendeln aufdestilliert, wobei folgende Fraktionen erhalten wurden: 1400 g Methanol (64 °C bis 68 °C, 950 mbar); 105 g Phenol (69 °C bis 72 °C, 12 mbar); und 5 g BF₃-Methanolkomplex (76 °C bis 80 °C, 12 mbar).

## Patentansprüche

1. Verfahren zur Reinigung eines unter Polyisobutenylphenolen mit einem zahlenmittleren Molekulargewicht von 200 bis 4000 oder Mannich-Addukten davon ausgewählten Phenolderivats, bei dem man das Phenolderivat mit einem Extraktionsmittel einer Polarität E_{T}(30) von 57 bis 38 kcal/mol innig in Kontakt bringt, eine Phenolderivat-haltige Phase und eine Extraktionsmittelphase sich voneinander trennen lässt und die Extraktionsmittelphase entfernt.

2. Verfahren nach Anspruch 1, wobei das Extraktionsmittel eine Polarität E_{T}(30) von 56 bis 48 kcal/mol aufweist.

3. Verfahren nach Anspruch 1 oder 2, bei dem man das Phenolderivat in einem Lösungsmittel mit einer Polarität E_{T}(30) von 35 bis 30 kcal/mol löst und die Lösung mit dem Extraktionsmittel in Kontakt bringt.

4. Verfahren nach einem der Ansprüche 1 bis 3, wobei das Extraktionsmittel unter Methanol, Ethanol, n-Propanol, Isopropanol, Aceton, Butanon oder Gemischen davon oder Gemischen davon mit Wasser ausgewählt ist.

5. Verfahren nach einem der vorhergehenden Ansprüche, bei dem das Polyisobutenylphenol ein zahlenmittleres Molekulargewicht von 500 bis 1500 aufweist.

6. Verfahren nach einem der vorhergehenden Ansprüche, bei dem das Mannich-Addukt das Umsetzungsprodukt des Polyisobutenylphenols mit Formaldehyd und einem primären oder sekundären Amin ist.

7. verfahren nach einem der vorhergehenden Ansprüche, bei dem es sich bei dem Polyisobutenylphenol um ein 4-Polyisobutenylphenol handelt.

8. Verfahren nach einem der vorhergehenden Ansprüche, bei dem man das Extraktionsmittel dem Phenolderivat in einer Extraktionszone entgegen führt.

## Claims

1. A process for purifying a phenol derivative selected from polyisobutenylphenols having a number average molecular weight of from 200 to 4000 or Mannich adducts thereof, in which the phenol derivative is brought into intimate contact with an extracting agent having a polarity E_{T}(30) of from 57 to 38 kcal/mol, a phase containing the phenol derivative and an extracting agent phase are allowed to separate from one another and the extracting agent phase is removed.

2. The process according to claim 1, wherein the extracting agent has a polarity E_{T}(30) of from 56 to 48 kcal/mol.

3. The process according to claim 1 or 2, in which the phenol derivative is dissolved in a solvent having a polarity E_{T}(30) of from 35 to 30 kcal/mol and the solution is brought into contact with the extracting agent.

4. The process according to any of claims 1 to 3, wherein the extracting agent is selected from methanol, ethanol, n-propanol, isopropanol, acetone, butanone and mixtures thereof and mixtures thereof with water.

5. The process according to any of the preceding claims, in which the polyisobutenylphenol has a number average molecular weight of from 500 to 1500.

6. The process according to any of the preceding claims, wherein the Mannich adduct is the reaction product of the polyisobutenylphenol with formaldehyde and a primary or secondary amine.

7. The process according to any of the preceding claims, in which the polyisobutenylphenol is a 4-polyisobutenylphenol.

8. The process according to any of the preceding claims, in which the extracting agent is passed countercurrently to the phenol derivative in an extraction zone.

## Revendications

1. Procédé de purification d'un dérivé de phénol choisi parmi des polyisobuténylphénols ayant un poids moléculaire moyen numérique de 200 à 4000 ou des produits d'addition de Mannich de ceux-ci, dans lequel on met intimement en contact le dérivé de phénol avec un agent d'extraction d'une polarité E_{T}(30) de 57 à 38 kcal/mole, on laisse se séparer l'une de l'autre une phase contenant du dérivé de phénol et une phase à agent d'extraction et on élimine la phase à agent d'extraction.

2. Procédé suivant la revendication 1, dans lequel l'agent d'extraction présente une polarité E_{T}(30) de 56 à 48 kcal/mole.

3. Procédé suivant la revendication 1 ou 2, dans lequel on dissout le dérivé de phénol dans un solvant ayant une polarité E_{T}(30) de 35 à 30 kcal/mole et on met en contact la solution avec l'agent d'extraction.

4. Procédé suivant l'une des revendications 1 à 3, dans lequel l'agent d'extraction est choisi parmi du méthanol, de l'éthanol, du n-propanol, de l'isopropanol, de l'acétone, de la butanone ou leurs mélanges ou leurs mélanges avec de l'eau.

5. Procédé suivant l'une des revendications précédentes, dans lequel le polyisobuténylphénol présente un poids moléculaire moyen numérique de 500 à 1500.

6. Procédé suivant l'une des revendications précédentes, dans lequel le produit d'addition de Mannich est le produit de la réaction du polyisobuténylphénol avec du formaldéhyde et une amine primaire ou secondaire.

7. Procédé suivant l'une des revendications précédentes, dans lequel, pour ce qui concerne le polyisobuténylphénol, il s'agit d'un 4-polyisobuténylphénol.

8. Procédé suivant l'une des revendications précédentes, dans lequel l'agent d'extraction passe à contre-courant du dérivé de phénol dans une zone d'extraction.
